# EUROPÄISCHE PATENTSCHRIFT

(11) **EP 3 407 832 B1**
(45) Veröffentlichungstag und Bekanntmachung des Hinweises auf die Patenterteilung: **27.11.2019**
(21) Anmeldenummer: 17705778.3
(22) Anmeldetag: 20.01.2017
(51) Int. Cl.: A61F 2/07, A61F 2/06, A61F 2/91, A61F 2/82

(54) **STENT-GRAFT-SYSTEM ZUR KOPPELUNG VON STENT-GRAFT ALS STENT-GRAFT-SYSTEM**
STENT GRAFT SYSTEM FOR COUPLING STENT GRAFTS AS A STENT GRAFT SYSTEM
SYSTÈME STENT-GREFFON DE COUPLAGE D'UN STENT-GREFFON COMME SYSTÈME STENT-GREFFON

(30) Priorität: 25.01.2016 DE 102016101273
(43) Veröffentlichungstag der Anmeldung: 05.12.2018
(73) Patentinhaber: Christian-Albrechts-Universität zu Kiel, 24118 Kiel (DE)
(72) Erfinder: GROSS, Justus, 24235 Laboe (DE)
(74) Vertreter: Hansen, Jochen
(86) Internationale Anmeldenummer: PCT/DE2017/100030
(87) Internationale Veröffentlichungsnummer: WO 2017/129167

(56) Entgegenhaltungen:
- EP-A1- 1 847 237
- WO-A1-2005/034808
- WO-A1-2014/163957
- WO-A2-03/063729
- US-A1- 2010 241 218
- US-B1- 6 325 826

## Beschreibung

Die Erfindung betrifft ein Stent-Graft-System mit einem auf einen Durchmesser bezogenen expandierbaren ersten Stent-Graft und mindestens einem zweiten Stent-Graft, wobei der erste Stent-Graft zumindest Bereichsweise, eine schlauchförmige Netzstruktur aufweist, die in einem expandierten Modus eine Netzstruktur mit im Wesentlichen kreisrunden, ringförmigen Maschen aufweist, wobei der mindestens zweite Stent-Graft an einem distalen Ende nach außen stehende Widerhaken aufweist, über die der mindestens zweite Stent-Graft an den ersten Stent-Graft im expandierten Modus ankoppelbar ist.

Im **Stand der Technik** erfolgt bei klassischen Verfahren, die derartige Prothesen miteinander verbinden, die Verbindung des Stent-Graft-Systems vor dem Einbringen in das Blutgefäß. Nachträgliche Änderungen oder Anpassungen vor Ort sind nur sehr eingeschränkt möglich.

Die Druckschrift US 2010/241218 A1 offenbart ein Stent-Graft-System mit einem auf einen Durchmesser bezogenen expandierbaren ersten Stent-Graft und mindestens einen zweiten Stent-Graft, wobei der erste Stent-Graft eine schlauchförmige Netzstruktur aufweist, wobei der mindestens zweite Stent-Graft an einem distalen Ende nach außen stehenden Widerhaken aufweist, über die der mindestens zweite Stent-Graft an den ersten Stent-Graft im expandierten Modus ankoppelbar ist, wobei der mindestens zweite Stent-Graft mit dem distalen Ende mit den jeweiligen nach außen stehenden Widerhaken an einer der kreisrunden, ringförmigen Fenestrierung eingreift.

Ein wesentliches **Problem im Stand der Technik** bzw. ein wesentlicher Nachteil der derzeit bekannten jeweiligen Sonderanfertigung einer solchen vor dem Einbringen bereits fertiggestellten vollständigen Prothese ist die fertige Geometrie, die mit der tatsächlichen Lage der Gefäße und Gefäßeinmündungen häufig nicht übereinstimmt.

Auch ist der Entfaltungsvorgang einer Prothese mit einer Reihe von Nebenabgängen die ergänzend gefaltet und dann entfaltet und nur sehr eingeschränkt positioniert werden können von Nachteil.

Der vorliegenden Erfindung liegt die **Aufgabe** zugrunde, ein Stent-Graft-System anzugeben, was die Probleme des Standes der Technik überwindet und insbesondere die optimale Anpassung vor Ort ermöglicht, so dass ein optimaler Stent-Graft an Ort und Stelle für das Organ realisiert werden kann.

**Gelöst** wird diese Aufgabe mit einem Stent-Graft-System gemäß Hauptanspruch.

Das Stent-Graft-System weist einen mit einem auf einen Durchmesser bezogenen expandierbaren ersten Stent-Graft und mindestens einem zweiten Stent-Graft auf, wobei der erste Stent-Graft zumindest bereichsweise, eine schlauchförmige Netzstruktur aufweist, die in einem expandierten Modus eine Netzstruktur mit im Wesentlichen kreisrunden, ringförmigen Maschen aufweist, wobei der mindestens zweite Stent-Graft an einem distalen Ende nach außen stehenden Widerhaken aufweist, über die der mindestens zweite Stent-Graft an den ersten Stent-Graft im expandierten Modus ankoppelbar ist, wobei der mindestens zweite Stent-Graft mit dem distalen Ende eine mit seinem Durchmesser korrespondierende kreisrunde, ringförmige Masche durchstößt und mit den jeweiligen nach außen stehenden Widerhaken an der kreisrunden, ringförmigen Masche eingreift, so dass eine Verbindung realisiert ist.

Im Zusammenhang mit der Erfindung Stent-Graft-System wird unter dem Begriff "ABLE-Stent" (Arch Branched Laserassisted Endovascular - Stent) die Konstruktion eines erfinderischen Stent-Graft-Systems unter zu Hilfenahme eines Laser-Führungskatheters zur Koppelung des Systems verstanden.

Der "ABLE - Stent" (Arch Branched Laserassisted Endovascular - Stent), also die diesseitige Erfindung, stellt ein neues Stent-Produkt dar, wodurch damit ein neues Verfahren der endovaskulären, insbesondere auch laserassistierte Behandlungen von Aortenbogenpathologien, wie Aneurysma und A-Dissektionen unter Neuroprotektion mittels Herzlungenmaschine möglich sind.

Die Besonderheiten dieser Erfindung sind:
- komplette endovaskuläre Versorgung der distalen Aorta ascendens, des Aortenbogens bis in in die Aorta descendens reichend;
- über eine extrakorporale Blutzirkulation wir eine konstante, neuro(embo lie-)protektive Durchblutung der bds. A. carotis erreicht
- neuartiges Ring- Design im Bogenbereich ermöglicht eine laserbasierte, retrograde Prothesenperforation über die Aa. brachiales und die Aa.carotis communis und Stent- Einlage
- der ABLE- StentGraft kann unter Rücksichtnahme auf die Durchblutung der Koronararterien auch mit einer minimalinvasiven Aortenklappe kombiniert werden.

Weiter kann der erste Stent-Graft mit einer Schicht, insbesondere einem Polymer, wie insbesondere Polytetrafluorethylen (PTFE), überzogen sein. Ferner kann das ganze Stent-Graft-System mit PTFE überzogen sein.

Ferner kann ein Material der Netzstruktur des ersten Stent-Grafts eine Formgedächtnis-Legierung, bevorzugt Nitinol, aufweisen.

Weiter kann die Netzstruktur des ersten Stent-Grafts mit nichtresorbierbarem Nahtmaterial umgarnt sein.

Der Branched-Stent-Durchmesser kann mit einer minimalen Übergröße dem Lumen der "Kreise" (Gitterstruktur) angepasst sein.

Die Widerhaken an den distalen Enden des zweiten Stents können sich in einem fortlaufenden Faden-Cover der Nitinol-Verstrebungen verankern.

Die erfindungsgemäße Neuerung des Stent-Grafts ist insbesondere eine lamellenartige Nitinol-Netz-Konfiguration in dem Bereich, wo nachfolgend das supraaortale Branching stattfinden soll. Aufgrund der längs-ovalären Form lässt sich der Bereich des ABLE-Stent-Grafts ebenso wie die herkömmlichen "Scherengitter"-Formen unter mechanischem Zug elongieren und im maximalen Querdurchmesser maßgeblich verkleinern, was die Grundvoraussetzung eines endovaskulären Stent-Graft-Systemes darstellt.

Der Unterschied ist jedoch derjenige, dass sich nach Auslösen, d.h.Verkürzen des Stent-Grafts nicht wie üblich ein rautenförmiges, sondern kreisrundes Nitinolgerüst aufspannt. Die mathematisch/geometrische Anordnung der Kreiskonfigurationen stellt dann die Kopplungsplatte zu den von supraaortal antegrad einzubringenden Stent-Graft-Branches dar. Im "Branch-Bereich" wird das Nitinolgerüst mit nichtresorbierbarem geflochtenen Material "umgarnt", welches dann zum Widerlager für die nachfolgende Branch-Verankerung wird.

Ein entsprechendes, für die weitere Verwendung und Ausführung des erfindungsgemäßen Stent-Graft, nämlich dazugehöriges erstes Verfahren zur Koppelung von erfindungsgemäßen Stent-Grafts als Stent-Graft-System weist die Schritte auf:
- Einbringen des auf einen Durchmesser bezogenen expandierbaren ersten Stent-Graft in einem nicht expandiertem Modus in ein Blutgefäß,
- Expandieren des ersten Stent-Graft mit zumindest bereichsweiser Bildung von im Wesentlichen kreisrunden, ringförmigen Maschen in der Netzstruktur,
- Einbringen des mindestens zweiten Stent-Graft durch ein weiteres Blutgefäß bzw. mehrerer zweite Stent-Graft durch ein oder mehrere weitere Blutgefäße, indem das jeweilige distale Ende des jeweiligen zweiten Stent-Graft in Richtung des ersten Stent-Graft, bevorzugt durch einen jeweiligen Führungskatheter, bewegt wird,
- Durchstoßen einer jeweiligen im Wesentlichen kreisrunden, ringförmigen Maschen in der Netzstruktur des ersten Stent-Graft durch einen jeweiligen zweiten Stent-Graft mit seinem jeweiligen distalen Ende,
- Ankoppeln des jeweiligen distalen Endes an die jeweiligen im Wesentlichen kreisrunden, ringförmigen Maschen in der Netzstruktur des ersten Stent-Graft durch mechanisches hintergreifendes Ziehen und Eingreifen der Widerhaken in die jeweilige kreisrunde, ringförmige Masche.

Ein entsprechendes, für die weitere Verwendung und Ausführung des erfindungsgemäßen Stent-Graft, nämlich dazugehöriges zweites Verfahren zur Koppelung von erfindungsgemäßen Stent-Grafts als Stent-Graft-System weist die Schritte auf:
- Einbringen des auf einen Durchmesser bezogenen expandierbaren ersten Stent-Graft in einem nicht expandiertem Modus in ein Blutgefäß,
- Expandieren des ersten Stent-Graft mit zumindest bereichsweiser Bildung von im Wesentlichen kreisrunden, ringförmigen Maschen in der Netzstruktur,
- Einbringen des mindestens zweiten Stent-Graft durch ein weiteres Blutgefäß bzw. mehrerer zweite Stent-Graft durch ein oder mehrere weitere Blutgefäße, indem das jeweilige distale Ende des jeweiligen zweiten Stent-Graft in Richtung des ersten Stent-Graft, bevorzugt durch einen jeweiligen Laser-Führungskatheter, bewegt wird,
- Perforation der jeweiligen Schicht des ersten Stent-Graft durch den jeweiligen Laser-Führungskatheter,
- Durchstoßen einer jeweiligen im Wesentlichen kreisrunden, ringförmigen Maschen in der Netzstruktur des ersten Stent-Graft durch einen jeweiligen zweiten Stent-Graft mit seinem jeweiligen distalen Ende,
- Ankoppeln des jeweiligen distalen Endes an die jeweiligen im Wesentlichen kreisrunden, ringförmigen Maschen in der Netzstruktur des ersten Stent-Graft durch mechanisches, hintergreifendes Ziehen und Eingreifen der Widerhaken in die jeweilige kreisrunde, ringförmige Masche.

Ein entsprechendes, für die weitere Verwendung und Ausführung des erfindungsgemäßen Stent-Graft, nämlich dazugehöriges drittes Verfahren zur Koppelung von erfindungsgemäßen Stent-Grafts als Stent-Graft-System weist die Schritte auf:
- Einbringen des auf einen Durchmesser bezogenen expandierbaren ersten Stent-Graft in einem nicht expandiertem Modus in ein Blutgefäß,
- Expandieren des ersten Stent-Graft mit zumindest bereichsweiser Bildung von im Wesentlichen kreisrunden, ringförmigen Maschen in der Netzstruktur,
- Einbringen des mindestens zweiten Stent-Graft durch ein weiteres Blutgefäß bzw. mehrerer zweite Stent-Graft durch ein oder mehrere weitere Blutgefäße, indem das jeweilige distale Ende des jeweiligen zweiten Stent-Graft in Richtung des ersten Stent-Graft, bevorzugt durch einen jeweiligen Laser-Führungskatheter, bewegt wird,
- Perforation der jeweiligen Schicht des ersten Stent-Graft durch den jeweiligen Laser-Führungskatheter,
- Durchstoßen einer jeweiligen im Wesentlichen kreisrunden, ringförmigen Maschen in der Netzstruktur des ersten Stent-Graft durch einen jeweiligen zweiten Stent-Graft mit seinem jeweiligen distalen Ende,
- Ankoppeln des jeweiligen distalen Endes an die jeweiligen im Wesentlichen kreisrunden, ringförmigen Maschen in der Netzstruktur des ersten Stent-Graft durch mechanisches, hintergreifendes Ziehen und Eingreifen der Widerhaken in die jeweilige mit nichtresorbierbarem Nahtmaterial umgarnte kreisrunde, ringförmige Masche.

Bei einer bevorzugten Ausgestaltung des Anwendungsverfahrens kommt ein Laser-Führungskatheter für die Positionierung der zweiten Stent-Graft im Stent-Graft-System zum Einsatz, die es erlaubt bei der Koppelung der Module des Systems, zwischen dem ersten expandierten von einer Schicht überzogenen Stent-Graft und dem zweiten Stent-Graft, der diese Schicht durchdringen muss, eine laserbasierte, retrograde Prothesenperforation des ersten Stent-Graft vorzunehmen, die ansonsten mechanisch erfolgt.

Nachfolgend wird ein Ausführungsbeispiel der Erfindung anhand der beiliegenden Zeichnungen in der **Figurenbeschreibung** detailliert beschrieben, wobei diese die Erfindung erläutern sollen und nicht beschränkend zu werten sind:

Es zeigen:
- Fig. 1 bis 3: erfindungsgemäßes Stent-Graft-System im Ablauf des Einbaus;
- Fig. 4 bis 6: Detailansicht des distalen Endes des zweiten Stent-Graft beim Einbauvorgang;
- Fig. 7 und 8: Detailansicht des ersten Stent-Grafts vor und nach der Expansion;

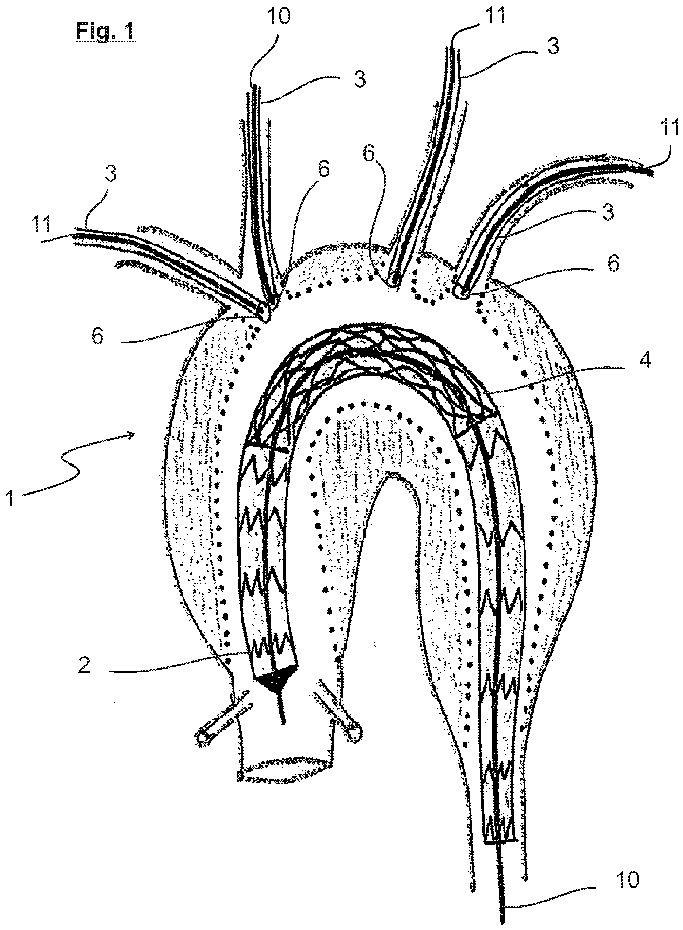

Die **Figuren 1 bis 3** zeigen einen Aortenbogen bei Aortenbogenaneurysma / A-Dissektion mit dem eingebrachten erfindungsgemäßen Stent-Graft-System 1.

Das Stent-Graft-System 1 besteht in diesem Ausführungsbeispiel aus einem ersten Stent-Graft 2 und einem zweiten Stent-Graft 3, wobei der erste Stent-Graft 2 in den Aortenbogen eingeführt ist.

Unter Durchleuchtung im Hybrid-OP wird auf Grundlage einer CT-Angiographie der Aortenbogen komplett dargestellt, so dass entsprechend gearbeitet werden kann.

Ablaufschritte:
- Vorschub des ABLE-Stent-Grafts 1 sowie der Laserkatheter supraaortal;
- bilateraler Zugang cervical zur A. carotis communis;
- bilateraler Zugang zur A. femoralis communis;
- Anlage eines arterio-arteriellen Shunts AFC -ACC bds., ggf. mit Flusskontrolle und extrakorpularer Zirkulationspumpe;
- offene antegrade Schleuseneinlage in die ACC bds. und Einbringen von Terumodrähten mit Laserkatheter, beispielsweise einem TurboElite 23 der Fa. Spectranetics, Colorado, USA an den Aortenbogen unter BV-Kontrolle;
- Punktion und antegrade Schleuseneinlage in die A. brachialis und Einbringen von Terumodrähten mit Laserkatheter an den Aortenbogen unter BV- Kontrolle;
- offene antegrade Schleuseneinlage unilateral femoral und unter BV Platzieren im Aortenbogen

Das Stent-Graft-System 1 wird entsprechend vervollständigt durch das Anbinden des zweiten Stent-Grafts 3 an den ersten Stent-Graft 2, indem sich die Widerhaken 7 des zweiten Stent-Grafts 3 in dem ersten Stent-Graft 2 verhaken.

Die einzelnen Elemente des Stent-Graft-Systems 1 sind der entsprechenden Bezugszeichenliste zu entnehmen.

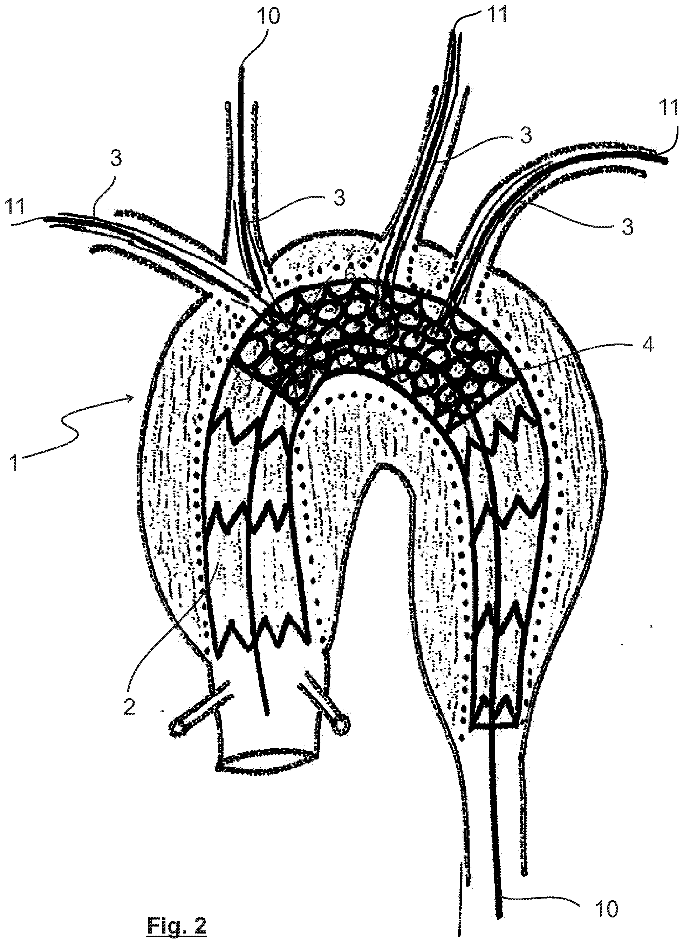

In **Fig. 2** ist dargestellt:
- das Entfalten des Aortenbogen-Stent-Grafts, dem ersten Stent-Graft 2, ggf. unter rapid Pacing;
- "Andocken" der Laserkatheter an den kreiskonfigurierten "branch"-Bereich, zufälliges Treffen einer Masche 5, beispielsweise einer Nitinol-Kreisspange, der Netzstruktur 4 und mittels niedriger Energie und hoher Repetitionsrate Laserperforation des PTFE-Covers des ersten Stent-Grafts 2;
- Drahtvorlage in das Lumen des Aortenbogens.

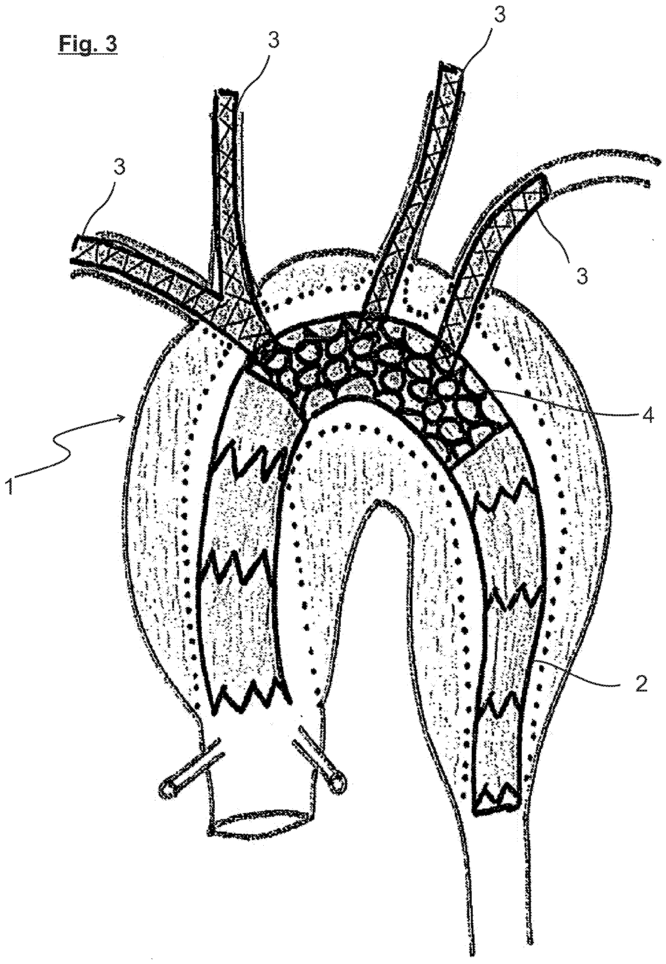

In **Fig. 3** ist die Dilatation der Grafts und das Beenden des Eingriffs dargestellt:
- Dilatation der Stent-Grafts;
- Übersichtangiographie;
- Aufgabe des Shunt/extrakorporale Zirkulation.

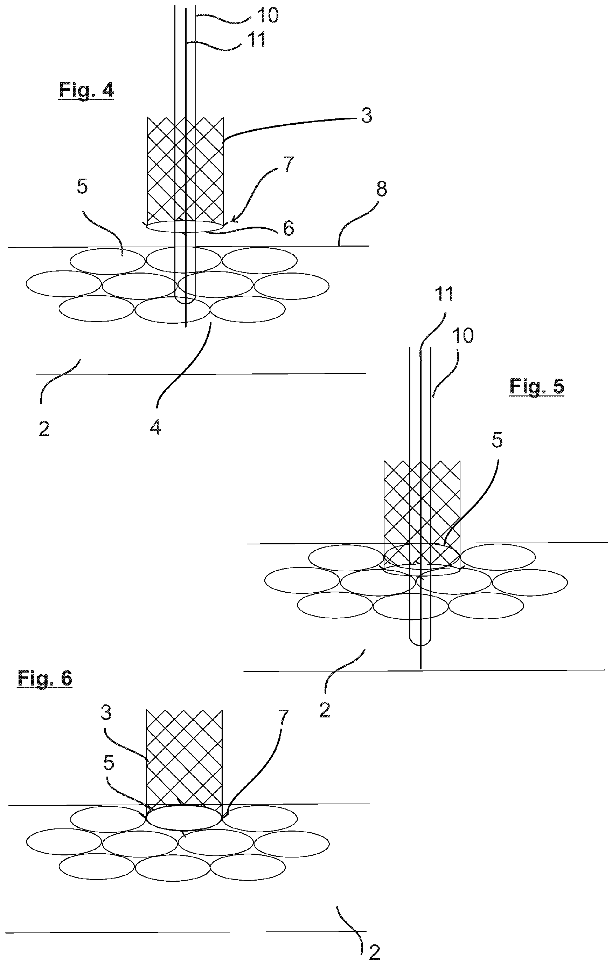

In den **Figuren 4 bis 6** ist der Einführ- und Verriegelungsvorgang des zweiten Stent-Grafts 3 in den ersten Stent-Graft 2 gezeigt.

Hierbei erfolgt ein laserassistiertes supraaortales Branching. Hierbei erfolgt die Applikation von handelsüblichen Branch-Stents in die ASL und ACC links, in den Truncus brachiocephalicus die Implantation eines "iliac-side-branch". Der Branched-Stent-Durchmesser ist mit minimalem oversizing dem Lumen der "Kreise" angepasst. Die Widerhaken 7 an den distalen Stent-Enden 6 verankern sich im fortlaufenden Faden-cover der beispielsweise Nitinol-Verstrebungen.

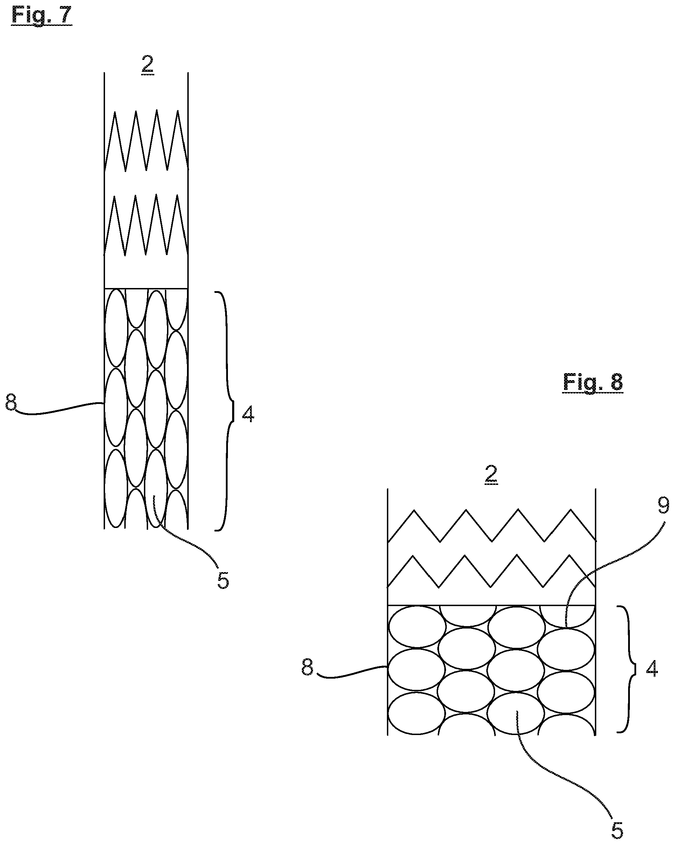

Die **Figuren 7 und 8** zeigen den ersten Stent-Grafts 2 vor der Entfaltung (Fig. 7) und nach der Entfaltung (Fig. 8). Es bilden sich im entfalteten Zustand wenigstens kreisähnliche Maschen 5 aus, die von dem zweiten Stent-Graft 3 durchstochen werden können.

### Bezugszeichenliste

- 1: Stent-Graft-System
- 2: erster Stent-Graft
- 3: zweiter Stent-Graft
- 4: Netzstruktur
- 5: Masche
- 6: distales Ende eines zweiten Stent-Graft
- 7: Widerhaken
- 8: Schicht
- 9: Nahtmaterial
- 10: Führungskatheter
- 11: Laser-Führungskatheter

## Patentansprüche

1. Stent-Graft-System (1) mit einem auf einen Durchmesser bezogenen expandierbaren ersten Stent-Graft (2) und mindestens einem zweiten Stent-Graft (3), wobei der erste Stent-Graft (2) zumindest Bereichsweise, eine schlauchförmige Netzstruktur (4) aufweist, die in einem expandierten Modus eine Netzstruktur (4) mit im Wesentlichen kreisrunden, ringförmigen Maschen (5) aufweist, wobei der mindestens zweite Stent-Graft (3) an einem distalen Ende (6) nach außen stehenden Widerhaken (7) aufweist, über die der mindestens zweite Stent-Graft (3) an den ersten Stent-Graft (2) im expandierten Modus ankoppelbar ist,
**dadurch, dass**
der mindestens zweite Stent-Graft (3) mit dem distalen Ende (6) eine mit seinem Durchmesser korrespondierende kreisrunde, ringförmige Masche (5) durchstößt und mit den jeweiligen nach außen stehenden Widerhaken (7) an der kreisrunden, ringförmigen Masche (5) eingreift.

2. Stent-Graft-System (1) nach Anspruch 1,
**dadurch gekennzeichnet, dass**
der erste Stent-Graft (2) mit einer Schicht (8), insbesondere einem Polymer, wie Polytetrafluorethylen (PTFE), überzogen ist.

3. Stent-Graft-System nach einem der vorangegangenen Ansprüche 2,
**dadurch gekennzeichnet, dass**
das ein Material der Netzstruktur (4) des ersten Stent-Graft (2) eine Formgedächnis Legierung, bevorzugt Nitinol, aufweist.

4. Stent-Graft-System nach einem der vorangegangenen Ansprüche,
**dadurch gekennzeichnet, dass**
das die Netzstruktur (4) des ersten Stent-Graft (2) mit nichtresorbierbarem Nahtmaterial (9) umgarnt ist.

## Claims

1. A stent graft system (1) having a first stent graft (2), expandable in diameter, and at least one second stent graft (3), wherein at least regions of the first stent graft (2) have a hose-shaped mesh structure (4) which in an expanded mode has a mesh structure (4) with substantially circular, annular loops (5), wherein the at least second stent graft (3) has outwards projecting barbs (7) at a distal end (6) via which the at least second stent graft (3) can be coupled to the first stent graft (2) in the expanded mode, **characterized in that** the distal end (6) of the at least second stent graft (3) penetrates a circular, annular loop (5) of corresponding diameter and engages the circular, annular loop (5) with the respective outwards projecting barbs (7).

2. A stent graft system (1) according to claim 1, **characterized in that** the first stent graft (2) is covered with a layer (8), in particular a polymer, such as polytetrafluorethylen (PTFE).

3. A stent graft system according to any one of the preceding claim 2 [sic], **characterized in that** a material of the mesh structure (4) of the first stent graft (2) has an alloy with shape memory, preferably nitinol.

4. A stent graft system according to any one of the preceding claims, **characterized in that** the mesh structure (4) of the first stent graft (2) is entwined with non-resorbable suture material (9).

## Revendications

1. Système de stent couvert (1) comprenant un premier stent couvert (2) dilatable en fonction d'un diamètre et au moins un deuxième stent couvert (3), le premier stent couvert (2) étant muni au moins par endroit d'une structure maillée (4) tubulaire qui présente, dans un mode dilaté, une structure maillée (4) ayant des mailles annulaires (5) sensiblement circulaires, ledit au moins deuxième stent couvert (3) étant muni à une extrémité distale (6) de barbes (7) s'étendant vers l'extérieur, par l'intermédiaire desquels ledit au moins deuxième stent couvert (3) peut être couplée au premier stent couvert (2) en mode dilaté,
**caractérisé en ce que**
ledit au moins deuxième stent couvert (3) perce avec l'extrémité distale (6) une maille annulaire circulaire (5) correspondant à son diamètre et s'engage sur la maille annulaire circulaire (5) avec chacune des barbes (7) situées à l'extérieur.

2. Système de stent couvert (1) selon la revendication 1,
**caractérisé en ce que**
le premier stent couvert (2) est revêtu d'une couche (8), en particulier d'un polymère comme le polytétrafluoroéthylène (PTFE).

3. Système de stent couvert (1) selon l'une des revendications précédentes 2, **caractérisé en ce que**
l'un des matériaux de la structure maillée (4) du premier stent couvert (2) comprend un alliage à mémoire de forme, de préférence du nitinol.

4. Système de stent couvert (1) selon l'une des revendications précédentes, **caractérisé en ce que** la structure maillée (4) du premier stent couvert (2) est recouverte d'un matériau de suture non résorbable (9).
